# EUROPEAN PATENT SPECIFICATION

(11) **EP 0 861 077 B1**
(45) Date of publication and mention of the grant of the patent: **06.02.2002**
(21) Application number: 96940390.6
(22) Date of filing: 12.11.1996
(51) Int. Cl.: A61K 31/44, A61K 31/455, A61K 31/465, C07D 401/10, C07D 401/12, C07D 405/14, C07D 213/81

(54) **HEMOREGULATORY COMPOUNDS**
HÄMOREGULATORISCHE VERBINDUNGEN
COMPOSES HEMOREGULATEURS

(30) Priority: 13.11.1995 US 6571 P; 13.11.1995 US 6573 P
(43) Date of publication of application: 02.09.1998
(73) Proprietor: SMITHKLINE BEECHAM CORPORATION, King of Prussia, PA 19406-0939 (US); Nycomed Austria GmbH, 4021 Linz (AT)
(72) Inventor: BHATNAGAR, Pradip, Kumar, Exton, PA 19341 (US); HARTMANN, Michael, A-4643 Pettenbach (AT); HIEBL, Johann, A-4030 Linz (AT); KREMMINGER, Peter, A-4481 Asten (AT); ROVENSZKY, Franz, A-4020 Linz (AT); Callahan, James Francis, PA 19111 Philadelphia (US); HEERDING, Dirk Andres, Malvern, PA 19355 (US)
(74) Representative: Walker, Ralph Francis, Dr.
(86) International application number: US9618126
(87) International publication number: WO9717964

(56) References cited:
- EP-A- 0 112 656
- WO-A-94/27627
- US-A- 3 917 626
- US-A- 4 544 661
- US-A- 5 360 806
- CHEMICAL ABSTRACTS, vol. 118, no. 18, 3 May 1993 Columbus, Ohio, US; abstract no. 169706, XP002087529 & K.J. BOUCK ET AL.: MACROMOLECULES, vol. 26, no. 8, 1993, pages 2077-2084,
- PATENT ABSTRACTS OF JAPAN vol. 015, no. 252 (C-0844), 26 June 1991 & JP 03 081222 A (YOYHITOMI PHARMACEUT. IND., LTD.), 5 April 1991

## Description

The present invention relates to novel compounds which have hemoregulatory activities and can be used to stimulate hematopoiesis and for the treatment of viral, fungal and bacterial infectious diseases.

### Background of the Invention

The hematopoietic system is a life-long cell renewal process whereby a defined stem cell population gives rise to a larger population of mature, differentiated blood cells (Dexter TM. Stem cells in normal growth and disease. Br Med J 1987; 195:1192-1194) of at least nine different cell lineages (erythrocytes, platelets, eosinophils, basophils, neutrophils, monocytes/macrophages, osteoclasts, and lymphocytes) (Metcalf D. The Molecular Control of Blood Cells. 1988; Harvard University Press, Cambridge, MA). Stem cells are also ultimately responsible for regenerating bone marrow following treatment with cytotoxic agents or following bone marrow transplantation.

The major dose-limiting toxicities of most standard anti-neoplastic drugs are related to bone marrow suppression, which if severe and prolonged, can give rise to life-threatening infectious and hemorrhagic complications. Myelosuppression is predictable and has been reported to be dose-limiting in greater than 50% of single-agent Phase I trials cytotoxic compounds (Merrouche Y, Catimel G, Clavel M., "Hematopoietic growth factors and chemoprotectants; should we move toward a two-step process for phase I clinical trials in oncology?" Ann Oncol 1993; 4:471-474). The risk of infection is directly related to the degree of myelosuppression as measured by the severity and duration of neutropenia (Brody GP, Buckley M, Sathe YS, Freireich EJ. "Quantitative relationship between circulating leukocytes and infections with acute leukemia." Ann In Med 1965; 64:328-334).

The control of hematopoiesis involves the interplay of a variety of cytokines and growth factors during various stages of the hematopoietic cascade, including early pluripotent stem cells and mature circulating effector cells. These regulatory molecules include granulocyte colony stimulating factor (G-CSF), granulocyte-macrophage stimulating factor (GM-CSF), macrophage-colony stimulating factor (M-CSF), and a variety of interleukins which have overlapping, additive and synergistic actions which play major roles in host defense. Mechanistically, this is accomplished by enhancing the production of granulocytes and macrophages, as well as by the activation of effector cell functions (Moore MAS. Hemopoietic growth factor interactions: *in vitro* and *in vivo* preclinical evaluation. Cancer Surveys 1990; 9:7-80). These coordinated activities support optimal host defences which are necessary for fighting bacterial, viral and fungal infections.

Strategies to prevent or reduce the severity of neutropenia and myelotoxicity include the use of hematopoietic growth factors and/or other hematopoietic cytokines. Such treatments are becoming common practice, in that they offer the potential of increased doses of cytotoxic agents that may improve the therapeutic efficacy of antineoplastic agents, and reduce the morbidity associated with their use (Steward WP. Granulocyte and granulocyte-macrophage colony stimulating factors, Lancet 1993; 342:153-157). Clinical studies have demonstrated the G-, GM- and/or M-CSF may reduce the duration of neutropenia, accelerate myeloid recovery, and reduce neutropenia-associated infections and other infectious complications in patients with malignancies who are receiving cytotoxic chemotherapy or in high infectious-risk patients following bone marrow transplantation (Steward WP. Granulocyte and granulocyte-macrophage colony stimulating factors, Lancet 1993; 342:153-157 and Munn DH, Cheung NKV. Preclinical and clinical studies of macrophage colony-stimulating factor. Semin Oncol 1992; 19:395-407).

Synthetic peptides have been reported to induce the synthesis and release of hematoporetic mediators, including m-CSF from bone marrow stromal elements see U.S. Patent Application 08/001,905.

We have now found certain novel non-peptide compounds which have a stimulative effect on myelopoietic cells. They are useful in stimulating myelopoiesis in patients suffering from reduced myelopoietic activity, including bone marrow damage, agranulocytosis and aplastic anemia including patients having depressed bone marrow function due to immunosuppressive treatment to suppress tissue reactions i.e. in bone marrow transplant surgery. They may also be used to promote more rapid regeneration of bone marrow after cytostatic chemotherapy and radiation therapy for neoplastic and viral diseases. They may be of particular value where patients have serious infections due to a lack of immune response following bone marrow failure. They are also useful in the treatment and prevention of viral, fungal and bacterial disease.

### Summary of the Invention

This invention comprises compounds, hereinafter represented as Formula (I), which have hemoregulatory activities and can be used to stimulate bematopoiesis and in the prevention and treatment of bacterial, viral and fungal diseases.

These compounds are useful in the restoration of leukocytes in patients with lowered cell counts resulting from a variety of clinical situations, such as surgically induced myelosuppression, AIDS, ARDS, congenital myelodysplacia, bone marrow and organ transplants; in the protection of patients with leukopenia from infection; in the treatment of severely burned patients and in the amelioration of the myelosuppression observed with some cell-cycle specific antiviral agents and in the treatment of infections in patients who have had bone marrow transplants, especially those with graft versus host disease, in the treatment of tuberculosis and in the treatment of fevers of unknown origin in humans and animals. The compounds are also useful in the treatment and prevention of viral, fungal and bacterial infectious diseases, particularly Candidiasis and Herpes in both immunosuppressed and "normal" subjects. They are useful in the treatment of sepsis caused by gram negative and gram positive organisms.

These compounds may also be used in combination with the myelosuppresive agents of co-pending U.S. Application No. 07/799,465 and U.S. Patent No. 4,499,081, incorporated by reference herein, to provide alternating peaks of high and low activity in the bone marrow cells, thus augmenting the natural circadian rhythm of hematopoiesis. In this way, cytostatic therapy can be given at periods of low bone marrow activity, thus reducing the risk of bone marrow damage, while regeneration will be promoted by the succeeding peak of activity. This invention is also a pharmaceutical composition, which comprises a compound of Formula (I) and a pharmaceutically acceptable carrier.

This invention further constitutes a method for stimulating the myelopoietic system of an animal, including humans, which comprises administering to an animal in need thereof, an effective amount of a compound of Formula (I).

This invention also constitutes a method for preventing and treating viral, fungal and bacterial infections in immunosuppressed and normal animals, including humans, which comprises administering to an animal in need thereof, an effective amount of a compound of Formula (I).

This invention further constitutes a method for preventing and treating sepsis caused by gram positive and gram negative organisms in animals, including humans, which comprises administering to an animal in need thereof, an effective amount of a compound of Formula (I).

### Detailed Description of the Invention

The compounds of the invention are represented by structural Formula (I) 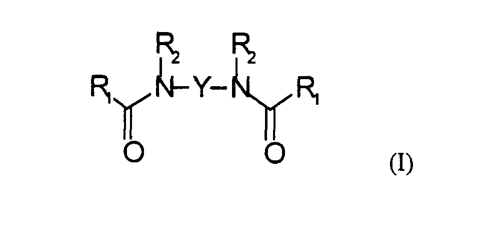 wherein:
R₁ is independently a 4 - 10 membered mono- or bicyclic heterocyclic ring system containing up to four heteroatoms N, O, S in the ring in which at least one heteroatom is N, and wherein the ring is substituted or unsubstituted by one or two C₁₋₄ alkyl, F, Cl, Br, I, C₁₋₄ alkoxy, (CH₂)ₘR₆, oxo, oxime, O-C₁₋₄alkyloxime, hydroxy, N(R₅)₂, acylamino or aminoacyl groups, 8, 9, 10 membered monocyclic ring systems being excluded;
R₂ is independently hydrogen, C₁₋₄alkylC(O)R₆, C₁₋₄alkyl or R₂ is benzyl which is optionally substituted by one or two C₁₋₄alkyl, C₁₋₄alkoxy, F, Cl, I, Br, OH, or N(R₅)₂;
Y is (CH₂)ₙ, in which at least one carbon is gem-substituted by R₃ and R₄;
R₃ and R₄ are independently C₁₋₄alkyl, C₂₋₄alkenyl, C₂₋₄alkynyl; all of which may be substituted by one or two C₁₋₄alkyl, OH, F, Cl, Br, I, N(R₅)₂, (R₅)₂NC(O)-,-(CH₂)ₘR₆,
   -(CH₂)ₘ R₅, -(CH)₂)ₘCOR₆, or -(CH₂)ₘC(O)R₅;
   or R₃ and R₄ are F, Cl, or Br;
   or R₃ and R₄ may together form a cyclic or heterocyclic ring of Formula (Ia): 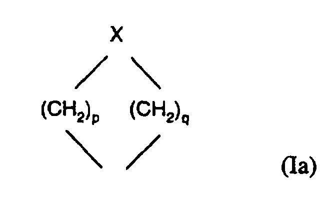 in which p and q are independently an integer from 0 to3;
   X is O, S, CH₂ or N(R₅);
   R₅ is independently hydrogen, C₁₋₄alkyl, or benzyl;
   R₆ is independently OR₅, N(R₅)₂ or SR₅; and
   n is an integer from 3 to 8;
   m is an integer from 0 to 4;
   or a pharmaceutical acceptable salt thereof;
   provided:
   p and q are not both 0; and
   when R₃ and R₄ are F, Cl or Br, they are not on a carbon adjacent to the nitrogen.

C₁₋₄ alkyl groups may be straight or branched.

The compounds of the present invention may contain one or more asymmetric carbon atoms and may exist in racemic and optically active form. All these compounds and diastereomers are contemplated to be within the scope of the present invention.

R₁ in the above Formula (I) denotes an optionally substituted pyrrolyl, isopyrrolyl, pyrazolyl, isoimidazolyl, triazolyl, isoxazolyl, oxazolyl, thiazolyl, isothiazolyl, oxadiazolyl, pyridinyl, pyridazinyl, pyrimidinyl, pyrazinyl, pyrrolidinyl, piperazinyl, triazinyl, morpholinyl, indolyl, indoleninyl, isobenzazolyl, pyrindinyl, isoindazolyl, indoxazinyl, benzoxazolyl, quinolinyl, isoquinolinyl, cinnolinyl, quinazolinyl, naphthyridinyl, pyridopyridinyl, tetrahydroquinolinyl, tetrahydroisoquinolinyl, quinoxalinyl, indolinyl, 2-pyrrolidonyl, imidazolyl, imidazolidinyl, imidazolinyl, piperidyl, tetrazolyl, quinuclidinyl, azetidinyl, or purinyl;

Preferred compounds are those wherein R₁ is pyridinyl, pyrimidinyl, pyrazinyl, pyridazinyl, quinolinyl, tetrahydroquinolinyl, azetidinyl, or pyrrolidinyl; n is 3-5; R₃ and R₄ are C₁₋₄alkyl, substituted by C₁₋₄ alkyl, OH, N(R₅)₁₋₂, -(CH₂)ₘR₆, or -(CH₂)ₘC(O)R₅; or R₃ and R₄ may together form a cyclic or heterocyclic ring of Formula (Ia) wherein X is O, S, or CH₂; and p and q are 1-3; R₅ is hydrogen or C₁₋₄alkyl; and R₆ is OR₅ or N(R₅)₁₋₂.

More preferred compounds are those wherein R₁ is 2-pyridinyl, 2-pyrimidinyl, 2-pyrazinyl, 2-pyrrolidon-5-yl, or 2-pyrrolidinyl; n is 3 or 5; R₃ and R₄ are C₁₋₂ alkyl, substituted by C₁₋₂alkyl, OH, N(R₅)₂, or -(CH₂)ₘR₆; or R₃ and R₄ may together form a heterocyclic ring of Formula (Ia) wherein X is O; and p and q are 1 or 2; R₅ is hydrogen or C₁₋₄alkyl; and R₆ is OR₅, or N(R₅)₁₋₂.

Preferred compounds are:
3,3-Bis(picolinamidomethyl)oxetane;
4,4-Bis(picolinamidomethyl)tetrahydro-4H-pyran;
2,2- Bis (picolinoylamidomethyl) 1,3 propanediol;
N,N'-Bis(picolinoyl)-1,5-diamino-2,2-dimethylpropane;
N,N'-Bis(picolinoyl)-1,5-diamino-3,3-dimethylpentane; and
1,1-Bis(picolinamidomethyl)cyclopropane.

The invention also is a pharmaceutical composition comprising as an active ingredient of one or more compounds of Formula (I) in association with a pharmaceutical carrier or excipient.

The invention is also a method of stimulating myelopoiesis in an animal, including humans, in need thereof by administering an effective amount of a compound of Formula I.

The invention is further a method of preventing or treating viral, fungal or bacterial infections comprising administering to an animal, invcluding humans, in need thereof, an effective amount of a compound of Formula (I).

### Methods of Preparation

Compounds of Formula (I) 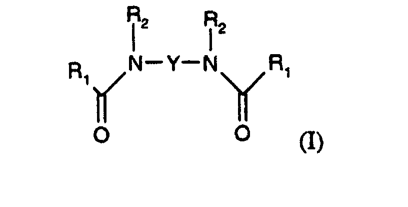 wherein R₁, R₂ and Y are as defined for Formula(I), are prepared as outlined in Scheme I. 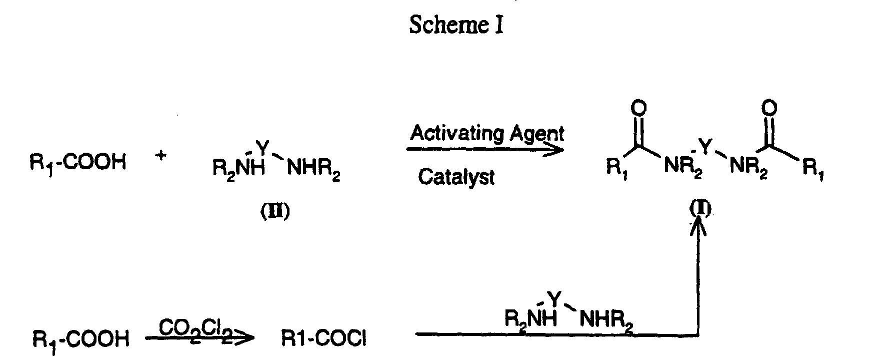 by reacting a suitable activated acid (R₁-COOH) with a suitable diamine (R₂-NH-Y-NH-R₂) or its salt form in a suitable solvent such as methylene chloride (CH₂Cl₂) or N,N-dimethylformamide (DMF) to give the final product. The carboxylic acid is activated with a suitable activating agent such as 1,3-dicylohexylcarbodiimide (DCC), benzotriazol-1-yloxy-tris(dimethylamino)phosphonium hexafluorophophate (BOP reagent), or 2-(1,H-benzotriazol-1-yl)-1,1,3,3, tetramethyluronium tetrafluoroborate (TBTU reagent). The reaction is catalyzed by a suitable catalyst such as 1-hydroxybenzotriazole hydrate (HOBt) or 4-dimethylaminopyridine (DMAP). If the amine is in its salt form, it must be neutalized by a suitable base such as triethylamine, Hunig's bas,e or N-methyl morpholine.

Alternatively, the acid is transformed to an acid halide that is reacted with an amine in the presence of a suitable base such as triethylamine, Hunig's base, or N-methyl morpholine, in a suitable solvent such as CH₂Cl₂, Benzene, or DMF to form the compound of Formula (I).

The amines of formula (II) (R₂-NH-Y-NH-R₂) may be obtained as shown in Scheme II 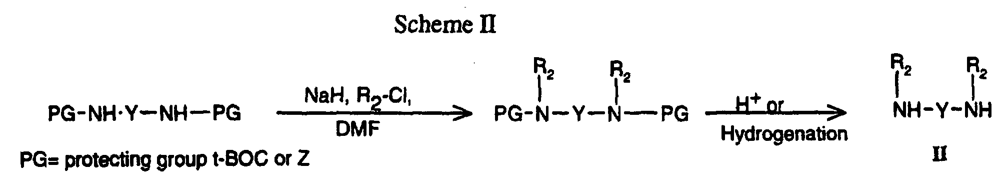 by reacting an appropriately protected diamine (NH₂-Y-NH₂) with an alkyl halide in the presence of base such as NaH. The reaction can be carried out in dioxane or DMF. The most commonly used protecting groups are t-butyloxycarbonyl (t-Boc-) or carbobenzyloxy (Z-). The methods of removing these protecting groups are well known in the art.

The amines of Formula (II) wherein R₂ is C₂₋₄ alkyl or C₂₋₄alkylC(O)R₆ may also be prepared as shown in Scheme (III) 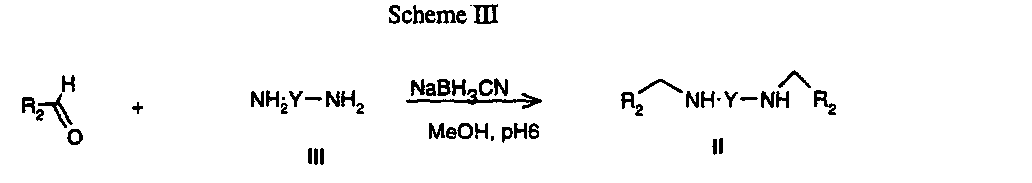 by reacting amines of formula III or IV with an aldehyde (R2-CHO) and a suitable reducing agent such as Sodium cyanoborohydride in a suitable solvent such as methanol.

The amines of Formula III (NH₂-Y-NH₂) can be obtained commercially or are prepared by reacting a suitable bisalkylhalide with ammonia.

The amines of Formula III or IV may also be prepared by reducing bisalkylnitiles or bisalkylazides as shown in Scheme IV 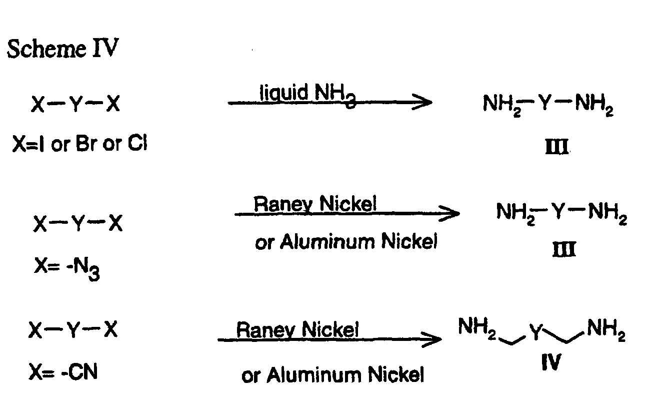 The compounds of Formula VI are prepared as shown in Scheme V 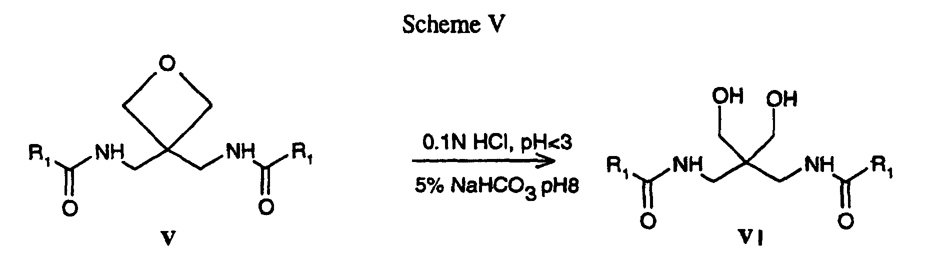 by sequentially reacting compounds of Formula V with an aqueous acid and aqueous base to give the desired compounds.

In order to use a compound of the Formula (I) or a pharmaceutically acceptable salt thereof for the treatment of humans and other mammals it is normally formulated in accordance with standard pharmaceutical practice as a pharmaceutical composition.

The compositions according to the invention may be presented for example, in a form suitable for oral, nasal, parenteral or rectal administration.

As used herein, the term "pharmaceutical" includes veterinary applications of the invention. These compounds may be encapsulated, tableted or prepared in an emulsion or syrup for oral administration. Pharmaceutically acceptable solid or liquid carriers may be added to enhance or stabilize the composition, or to facilitate preparation of the composition. Liquid carriers include syrup, peanut oil, olive oil, glycerin, saline and water. Solid carriers include starch, lactose, calcium sulfate dihydrate, terra alba, magnesium stearate or stearic acid, talc, pectin, acacia, agar or gelatin. The carrier may also include a sustained release material such a glyceryl monostearate or glyceryl distearate, alone or with a wax. The amount of solid carrier varies but, preferably will be between about 20 mg to about 1 g per dosage unit. The pharmaceutical preparations are made following the conventional techniques of pharmacy involving milling, mixing, granulating, and compressing, when necessary, for tablet forms; or milling, mixing and filling for hard gelatin capsule forms. Capsules containing one or several active ingredients may be produced, for example, by mixing the active ingredients with inert carriers, such as lactose or sorbitol, and filling the mixture into gelatin capsules. When a liquid carrier is used, the preparation will be in the form of a syrup, elixir, emulsion or an aqueous or non-aqueous suspension. Such a liquid formulation may be administered directly p.o. or filled into a soft gelatin capsule. Organ specific carrier systems may also be used.

Alternately pharmaceutical compositions of the compounds of this invention, or derivatives thereof, may be formulated as solutions of lyophilized powders for parenteral administration. Powders may be reconstituted by addition of a suitable diluent or other pharmaceutically acceptable carrier prior to use. The liquid formulation is generally a buffered, isotonic, aqueous solution. Examples of suitable diluents are normal isotonic saline solution, standard 5% dextrose in water or buffered sodium or ammonium acetate solution. Such formulation is especially suitable for parenteral administration, but may also be used for oral administration and contained in a metered dose inhaler or nebulizer for insufflation. It may be desirable to add excipients such as polyvinylpyrrolidone, gelatin, hydroxy cellulose, acacia, polyethylene glycol, mannitol, sodium chloride or sodium citrate.

For rectal administration, a pulverized powder of the compounds of this invention may be combined with excipients such as cocoa butter, glycerin, gelatin or polyethylene glycols and molded into a suppository. The pulverized powders may also be compounded with an oily preparation, gel, cream or emulsion, buffered or unbuffered, and administered through a transdermal patch.

Nasal sprays may be formulated similarly in aqueous solution and packed into spray containers either with an aerosol propellant or provided with means for manual compression.

Dosage units containing the compounds of this invention preferably contain .05-50 mg, for example .05-5 mg of the compound of Formula (I) or salt thereof.

According to a still further feature of the present invention there is provided a method of stimulation of myelopoiesis which comprises administering an effective amount of a pharmaceutical composition of Formula (I) as hereinbefore defined to a subject.

No unacceptable toxicological effects are expected when compounds of the invention are administered in accordance with the present invention.

The biological activity of the compounds of Formula (I) are demonstrated by the following tests.

### Induction of Hematopoietic Synergistic Activity in Stromal Cells

The murine bone marrow derived stromal cell line, C6.4 is grown in 12 well plates in RPMI 1640 with 10% FBS. Upon reaching confluence, the C6.4 cells are washed and the media exchanged with fresh RPMI 1640 without FBS. Confluent cell layers of murine C6.4 cells are treated with compound. Cell-free supernatants are collected 18 hours later. Supernatants are fractionated with a Centricon-30 molecular weight cut-off membrane. C6.4 cell hematopoietic synergistic factor (HSF) activity is measured in a murine CFU-C assay.

### CFU-C Assay

Bone marrow cells are obtained from C57B1/6 female mice and suspended in RPMI 1640 with 10% FBS. Bone marrow cells (7.5E+4 cells/mL) are cultured with sub optimal levels of CFU plus dilutions of test C6.4 cell 30K-E supernatants from above in a standard murine soft agar CFU-C assay. Cell aggregates >50 cells are counted as colonies. The number of agar colonies counted is proportional to the amount of HSF present within the C6.4 bone marrow stromal line supernatant.

### Effector Cell Function Assay

Female C57B1 mice are administered test compound IP or PO daily for 8 days. Resident peritoneal exudate cells (PEC) utilized *ex vivo* from treated or untreated mice are harvested with cold calcium and magnesium-free DPBS supplemented with heparin and antibiotics within 2-4 hours following the last injection. Adherent PEM populations are prepared by incubating standardized PEC suspensions in microtiter dishes for 2 hours at 37 °C (5% CO₂) and removing nonadherent cells by washing the wells with warm buffer.

The superoxide dismutase-inhibitable (SOD) superoxide released byeffector cells in response to a *in vitro* stimulation by phorbol myristate acetate (PMA) (100-200nM) or pre-opsonized (autologous sera) · live *C*. *albicans* (E:T = 1:10) are quantitated in a microtiter ferricytochrome c reduction assay. The assay is performed in the presence of 1% gelatin/HBSS and 80uM ferricytochrome *c* in a total volume of 200uL/well. The nmoles of cytochrome *c* reduced/well is calculated from spectrophotometric readings (550 nm) taken following a 1 hour incubation at 37 °C (5% CO₂). The amount of SOD-inhibitable cytochrome *c* reduced is determined by the inclusion of wells containing SOD (200 U/well). Baseline superoxide release is determined in the absence of stimuli. Experimental data are expressed as a percentage of the control group.

The following examples are illustrative and are not limiting of the compounds of this invention.

All temperatures are given in Centigrade.

### EXAMPLE 1

### 3,3-Bis(picolinamidomethyl)oxetane

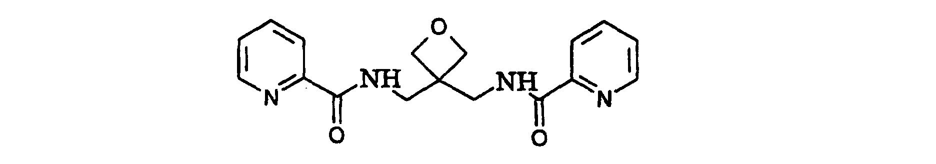

To a solution of 3,3-bisaminomethyloxetane dihydrobromide (0.05 g, 0.18 mmol) in DMF (3 mL) was added picolinic acid (0.07 g, 0.56 mmol), BOP reagent (0.24 g, 0.54 mmol), HOBt hydrate (0.07 g, 0.52 mmol) and iPr₂NEt (0.16 mL, 0.92 mmol). After 24 h, the reaction was quenched by pouring into 50% brine (10 mL) and extracting with EtOAc (3 x 15 mL). The combined organic extracts were dried over Na₂SO₄ and concentrated to give a yellow oil (0.82 g). Flash chromatography (gradient 50% EtOAc/ hexane to 100% EtOAc, silica gel) gave 0.02 g, (34%) of the above named product as a white solid. ¹H NMR (CDCl₃, 250 MHz) δ 8.82 (t, 2H, J=7 Hz), 8.61 (m, 2H), 8.23 (d,d, 2H, J=7.7, 1 Hz), 7.87 (d,t, 2H, J=7.7, 1.6 Hz), 7.45 (m, 2H), 4.59 (s, 4H), 3.87 (d, 4H, J=7Hz); MS (ES+) m/e 327.4 [M+H⁺].

### EXAMPLE 2

### 2,2- Bis (picolinoylamidomethyl) 1,3 propanediol;

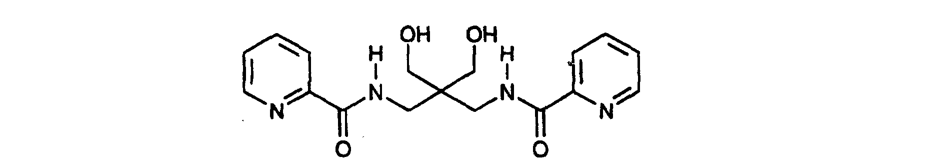

3,3-Bis(picolinamidomethyl)oxetane (example 1) (25.6 mg, 0.078 mmol) was dissolved in 8 mL of 0.1 N HCl (aqueous) and stirred at room temperature for 18 h. The reaction was made basic (pH~8) with 5% NaHCO₃ (aqueous) and evaporated to dryness. The residue was taken into 10% MeOH/CHCl₃, filtered and evaporated and the resulting material purified by flash chromatography (silica gel, 3x20 cm, 2% MeOH/CHCl₃) to give two fractions. The first fraction contained 5 mg(0.015 mmol, 19%) of recovered starting material. MSES m/e 327 (M+H)⁺. The second fraction contained 19.3 mg (0.056 mmol, 72%) of the above named product. ¹H NMR (CDCl₃, 250 MHz) δ 8.77 (t, 2H, J=7Hz), 8.61 (m, 2H), 8.21 (d,d, 2H, J= 7.8, 1 Hz), 7.88 (t,d, 2H, J= 7.8, 1.7 Hz), 7.47 (m, 2H), 4.44 (t, 1H, J= 7Hz), 3.48 (d, 4H, J=7Hz), 3.37 (d, 4H, J=7Hz); MS(ES+) m/e 345 [M+H]⁺.

### EXAMPLE 3

### 4,4-Bis(picolinamidomethyl)tetrahydro-4H-pyran

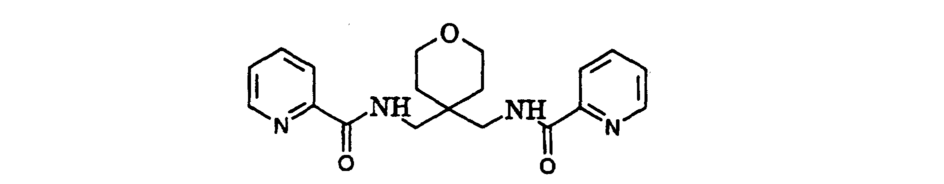

To a stirred mixture of tetrahydro-4H-pyran-4,4-dicarbonitrile (0.33g, 0.002mole) in MeOH (10ml) and 2N NaOH (9ml) at 0° was added in portions, aluminum-nickel catalyst (0.59g, 0.01mole). The reaction mixture was refluxed for 4 hours. The cooled mixture was adjusted to pH2 by dropwise addition of concentrated HCl. The mixture was then concentrated under reduced pressure at 40° to give an off-white solid.

To a solution of picolinic acid (0.59g, 0.0048mole) in benzene (25ml) and one drop of DMF was added under argon, oxalyl chloride (0.64g, 0.0048mole). The reaction was stirred at room temperature for 3 hours and then cooled to 0°. To this was added in one portion a suspension of the diamino-tetrahydropyran dihydrochloride salt of (0.35g, 0.0016mole) in chloroform (5ml) containing diisopropylethylamine (2.06g, 0.016mole). The reaction was stirred under argon at room temperature for 2 hours. The mixture was poured out into 50ml of brine and extracted twice with EtOAc. The EtOAc extracts were dried(Na₂SO₄), filtered and concentrated at 40° to a brown oil. The oil was flash chromatagraphed on silica-gel eluting with EtOAc to yield 22.5mg of the above named prodcut as a colorless oil. ¹H NMR(CDCl₃)δ: 8.9(broad t, 2H), 8.6(m, 2H), 8.3(m, 2H), 7.8(m, 2H), 7.5(m, 2H), 3.9(t, 4H), 3.5(d, 4H), 1.6(t, 4H);
MS (ES+) m/e 355 [M+H]⁺; 377 [M+H+Na]⁺.

### EXAMPLE 4

### 1,1-Bis(picolinamidomethyl)cyclopropane

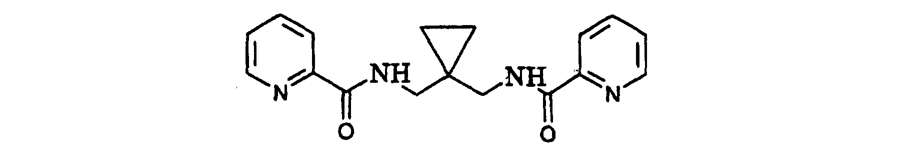

In a manner analogous to that of Example 3, 1,1-diaminomethylcycopropane (0.53 g, 3.09 mmol), picolinic acid (1.14 g, 9.26 mmol), BOP reagent (4.10 g, 9.26 mmol), HOBt hydrate (1.25 g, 9.25 mmol) and Et₃N (2.60 mL, 18.6 mmol) gave 0.83 g (87%) of the above named product as a white foam. MS (ES+) m/e 311.4 [M+H⁺].

### EXAMPLE 5

### N,N'-Bis(picolinoyl)-1,5-diamino-3,3-dimethylpentane

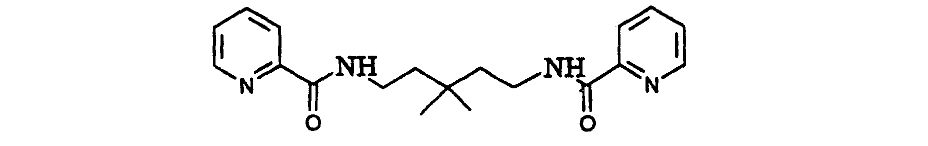

In a manner analogous to that of Example 1, 3,3-dimethyl-1,5-diaminopentane (0.22 g, 1.70 mmol), picolinic acid (0.46 g, 3.74 mmol), BOP reagent (1.65 g, 3.74 mmol), HOBt hydrate (0.50 g, 3.74 mmol), and iPr₂NEt (2.96 mL, 17.0 mmol) gave a yellow oil. Trituration from hot EtOAc gave 0.24 g (42%) of the above named product as white crystals. MS(ES+) m/e 341.0 [M+H⁺].

### EXAMPLE 6

### N,N'-Bis(picolinoyl)-1,3-diamino-2,2-dimethylpropane

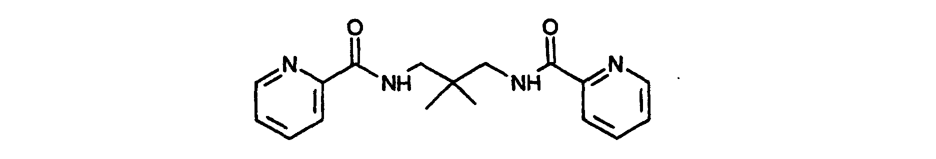

A solution of 2,2-dimethyl-1,3-propanediamine (5.00 g, 48.9 mmol) in DMF (500 mL) was treated with picolinic acid (12.6 g, 0.10 mol), 1-hydroxybenzotriazole hydrate (13.9 g, 0.10 mol), 1-(3-dimethylaminopropyl)-3-ethylcarbodiimide hydrochloride (19.7 g, 0.10 mol) and iPr₂NEt (17.9 mL, 0.10 mol) at room temperature. After 18 h, the reaction mixture was evaporated under vacuum and the residue purified by flash chromatography (75% EtOAc/hexanes, silica gel) to give 13.95 g (91%) the above named prodcut as a clear solid. A fraction of this product (5.63 g) was recrystallized from ethyl acetate/hexane by slow evaporation to dive 3.92 g of pure product as white needles. MS (ES+) m/e 313 [M+H⁺].

### EXAMPLE 7

Formulations for pharmaceutical use incorporating compounds of the present invention can be prepared in various forms and with numerous excipients. Examples of such formulations are given below.

| Tablets/Ingredients | Per Tablet |
|---|---|
| 1. Active ingredient (Cpd of Form. I) | 0.5 mg |
| 2. Corn Starch | 20 mg |
| 3. Alginic acid | 20 mg |
| 4. Sodium alginate | 20 mg |
| 5. Mg stearate | 1.3 mg |

### Procedure for tablets:

- Step 1: Blend ingredients No. 1, No. 2, No. 3 and No. 4 in a suitable mixer/blender.
- Step 2: Add sufficient water portion-wise to the blend from Step 1 with careful mixing after each addition. Such additions of water and mixing until the mass is of a consistency to permit its converion to wet granules.
- Step 3: The wet mass is converted to granules by passing it through an oscillating granulator using a No. 8 mesh (2.38 mm) screen.
- Step 4: The wet granules are then dried in an oven at 140°F (60°C) until dry.
- Step 5: The dry granules are lubricated with ingredient No. 5.
- Step 6: The lubricated granules are compressed on a suitable tablet press.

### Parenteral Formulation

A pharmaceutical composition for parenteral administration is prepared by dissolving an appropriate amount of a compound of Formula I in polyethylene glycol with heating. This solution is then diluted with water for injections Ph Eur. (to 100 ml). The solution is then sterilized by filtration through a 0.22 micron membrane filter and sealed in sterile containers.

## Claims

1. Compounds having the formula (I) 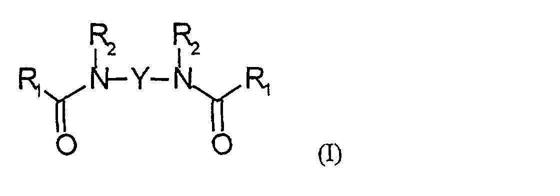 wherein:
R₁ is independently a 4 - 10 membered mono- or bicyclic heterocyclic ring system containing up to four heteroatoms N, O, S in the ring in which at least one heteroatom is N, and wherein the ring is substituted or unsubstituted by one or two C₁₋₄ alkyl, F, Cl, Br, I, C₁₋₄ alkoxy, (CH₂)ₘR₆, oxo, oxime, O-C₁₋₄alkyloxime, hydroxy, N(R₅)₂, acylamino or aminoacyl groups, 8, 9, 10 membered moncyclic ring sytems being excluded;
R₂ is independently hydrogen, C₁₋₄alkylC(O)R₆, C₁₋₄alkyl or R₂ is benzyl which is optionally substituted by one or two C₁₋₄alkyl, C₁₋₄alkoxy, F, Cl, I, Br, OH, or N(R₅)₂;
Y is (CH₂)ₙ, in which at least one carbon is gem-substituted by R₃ and R₄; R₃ and R₄ are independently C₁₋₄alkyl, C₂₋₄alkenyl, C₂₋₄alkynyl; all of which may be substituted by one or two C₁₋₄alkyl, OH, F, Cl, Br, I, N(R₅)₂, (R₅)₂NC(O)-,
-(CH₂)ₘR₆, -(CH₂)ₘ R₅, -(CH)₂)ₘCOR₆, or -(CH₂)ₘC(O)R₅;
or R₃ and R₄ are F, Cl, or Br;
or R₃ and R₄ may together form a cyclic or heterocyclic ring of Formula (Ia): 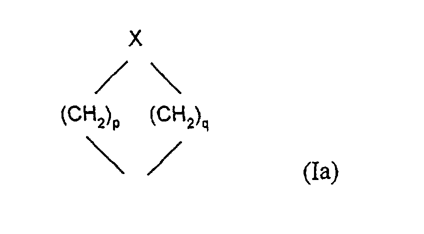 in which p and q are independently an integer from 0 to 3;
X is O, S, CH₂ or N(R₅);
R₅ is independently hydrogen, C₁₋₄alkyl, or benzyl;
R₆ is independently OR₅, N(R₅)₂ or SR₅; and
n is an integer from 3 to 8;
m is an integer from 0 to 4;
or a pharmaceutical acceptable salt thereof;
provided: p and q are not both 0; and when R₃ and R₄ are F, Cl or Br, they are not on a carbon adjacent to the nitrogen
and excluding N5, N5-'or N4, N4'-(2,2-dimethyl-1,3-propanediyl)bis[1-methyl-. 1H-Imidazole-4,5-dicarboxamide].

2. Compounds according to claim 1 wherein R₁ is pyridinyl, pyrimidinyl, pyrazinyl, pyridazinyl, quinolinyl, tetrahydroquinolinyl, or pyrrolidonyl; n is 3-5; R₃ and R₄ are C₁₋ ₄alkyl, substituted by C₁₋₄ alkyl, OH, N(R₅)₁₋₂, -(CH₂)ₘR₆, or -(CH₂)ₘC(O)R₅; or R₃ and R₄ may together form a cyclic or heterocyclic ring of Formula (Ia) wherein X is O, S, or CH₂; and p and q are 1-3; R₅ is hydrogen or C₁₋₄alkyl; and R₆ is OR₅ or N(R₅)₁₋₂.

3. Compounds according to claim 2 wherein R₁ is 2-pyridinyl, 2-pyrimidinyl, 2-pyrazinyl, 2-pyrrolidon-5-yl, or 2-pyrrolidinyl; n is 3 or 5; R₃ and R₄ are C₁₋₂ alkyl, substituted by C₁₋₂alkyl, OH, N(R₅)₂, or -(CH₂)ₘR₆; or R₃ and R₄ may together form a heterocyclic ring of Formula (Ia) wherein X is O; and p and q are 1 or 2; R₅ is hydrogen or C₁₋₄alkyl; and R₆ is OR₅, or N(R₅)₁₋₂.

4. Compounds according to claim 1 selected from the group consisting of 3,3-Bis(picolinamidomethyl)oxetane; 4,4-Bis(picolinamidomethyl)tetrahydro-4H-pyran; 2,2- Bis (picolinoylamidomethyl) 1,3 propanediol; N,N'-Bis(picolinoyl)-1,5-diamino-2,2-dimethylpropane; N,N'-Bis(picolinoyl)-1,5-diamino-3,3-dimethylpentane; and 1,1-Bis(picolinamidomethyl)cyclopropane.

5. A compound according to claim 1 which is N,N'-Bis(picolinoyl)-1,3-diamino-2,2-hydroxymethylpropane.

6. A pharmaceutical composition comprising a compound according to any one of claims 1 to 5 and a pharmaceutical carrier or excipient.

7. A compound according to any one of claims 1 to 5 for use in therapy.

8. Use of a compound according to any one of claims 1 to 5 in the manufacture of a medicament for use in stimulating the myelopoietic system of an animal.

9. Use of a compound according to any one of claims 1 to 5 in the manufacture of a medicament for use in the prevention or treatment of a viral, fungal or bacterial infection.

10. Use of a compound according to any one of claims 1 to 5 in the manufacture of a medicament for use in the prevention or treatment of sepsis.

11. A process for preparing a compound according to any one of claims 1 to 5 which comprises:
(a) Bisacylating a diamine of Formula (II):
HN(R₂)-Y-(R₂)NH Formula (II)
wherein R₂ and Y are as defined in Formula (I) or Y may additionally be protected by a suitable protecting group; with a compound of Formula (IV):
R₁-CO₂H Formula (IV)
wherein R₁ is as defined in Formula (I), using a suitable activating agent in a suitable polar aprotic solvent, followed by removal of any protecting groups and, if desired, by salt formation; or alternately by:
(b) transforming the acid of Formula (IV) to an acid halide:
R₁COX'
wherein R₁ is as defined in Formula (I), and X' is a halogen atom,
that is reacted with the diamine of Formula (II)
HN(R₂)-Y-(R₂)NH Formula (III)
wherein R₂ and Y are as defined in Formula (I) or Y may additionally be protected by a suitable protecting group; in the presence of a suitable base, in a suitable solvent, followed by removal of any protecting groups and, if desired, by salt formation.

## Patentansprüche

1. Verbindungen der Formel (I) 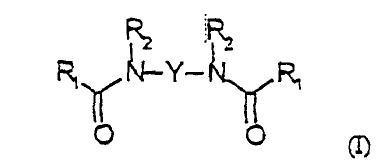 in der:
R₁ unabhängig für ein vier- bis zehngliedriges mono- oder bicyclisches heterocyclisches Ringsystem steht, das bis zu vier Heteroatome N, O, S in dem Ring enthält, wobei es sich bei mindestens einem Heteroatom um N handelt, und wobei der Ring unsubstituiert oder durch einen oder zwei C₁₋₄-Alkylreste, F, Cl, Br, I, C₁₋₄-Alkoxyreste, (CH₂)ₘR₆, eine Oxogruppe, Oximgruppe, einen O-C₁₋₄-Alkyloximrest, Hydroxygruppen, N(R₅)₂, Acylamino- oder Aminoacylgruppen substituiert ist, wobei 8-, 9-, 10-gliedrige monocyclische Ringsysteme ausgenommen sind;
R₂ unabhängig für ein Wasserstoffatom, einen Rest C₁₋₄-AlkylC(O)R₆, einen C₁₋₄-Alkylrest steht oder R₂ für eine Benzylgruppe steht, die gegebenenfalls durch einen oder zwei C₁₋₄-Alkylreste, C₁₋₄-Alkoxygruppen, F, Cl, I, Br, OH oder N(R₅)₂ substituiert ist;
Y für (CH₂)ₙ steht, wobei mindestens ein Kohlenstoff durch R₃ und R₄ geminal-substituiert ist;
R₃ und R₄ unabhängig für einen C₁₋₄-Alkylrest, C₂₋₄-Alkenylrest, C₂₋₄-Alkinylrest stehen, die alle durch einen oder zwei C₁₋₄-Alkylreste, OH, F, Cl, Br, I, N(R₅)₂, (R₅)₂NC(O)-, -(CH₂)ₘR₆, -(CH₂)ₘR₅, -(CH₂)ₘCOR₆ oder -(CH₂)ₘC(O)R₅ substiuiert sein können;
oder R₃ und R₄ für F, Cl oder Br stehen;
oder R₃ und R₄ zusammen einen cyclischen oder beterocyclischen Ring der Formel (Ia) bilden können: 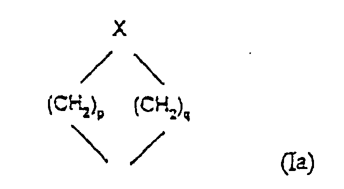
in der p und q unabhängig eine ganze Zahl von 0 bis 3 darstellen;
X für O, S, CH₂ oder N(R₅) steht;
R₅ unabhängig für ein Wasserstoffatom, einen C₁₋₄-Alkylrest oder eine Benzylgruppe steht;
R₆ unabhängig für OR₅, N(R₅)₂ oder SR₅ steht; und
n eine ganze Zahl von 3 bis 8 bedeutet;
m eine ganze Zahl von 0 bis 4 bedeutet;
oder ein pharmazeutisch verträgliches Salz davon;
mit der Maßgabe, daß nicht sowohl p als auch q 0 sind; und wenn R₃ und R₄ für F, Cl oder Br stehen, diese sich nicht an einem zu dem Stickstoffatom benachbarten Kohlenstoffatom befinden;
und unter Ausschluß von N5,N5'- oder N4,N4'-(2,2-Dimethyl-1,3-propandiyl)bis[1-methyl-1H-imidazol-4,5-dicarboxamid].

2. Verbindungen nach Anspruch 1, wobei R₁ für eine Pyridinyl-, Pyrimidinyl-, Pyrazinyl-, Pyridazinyl-, Chinolinyl-, Tetrahydrochinolinyl- oder Pyrrolidonylgruppe steht; n 3 bis 5 beträgt, R₃ und R₄ für einen C₁₋₄-Alkylrest stehen, der durch einen C₁₋₄-Alkylrest, OH, N(R₅)₁₋₂, -(CH₂)ₘR₆ oder -(CH₂)ₘC(O)R₅ substituiert ist; oder R₃ und R₄ zusammen einen cyclischen oder heterocyclischen Ring der Formel (Ia) bilden können, wobei X für O, S oder CH₂ steht; und p und q 1 bis 3 betragen; R₅ ein Wasserstoffatom oder einen C₁₋₄-Alkylrest bedeutet; und R₆ für OR₅ oder N(R₅)₁₋₂ steht.

3. Verbindungen nach Anspruch 2, wobei R₁ für eine 2-Pyridinyl-, 2-Pyrimidinyl-, 2-Pyrazinyl-, 2-Pyrrolidon-5-yl- oder 2-Pyrrolidinylgruppe steht; n 3 oder 5 beträgt; R₃ und R₄ für einen C₁₋₂-Alkylrest stehen, der durch einen C₁₋₂-Alkylrest, OH, N(R₅)₂ oder -(CH₂)ₘR₆ substituiert ist; oder R₃ und R₄ zusammen einen heterocyclischen Ring der Formel (Ia) bilden können, wobei X für O steht; und p und q 1 oder 2 betragen; R₅ für ein Wasserstoffatom oder einen C₁₋₄-Alkylrest steht; und R₆ für OR₅ oder N(R₅)₁₋₂ steht.

4. Verbindungen nach Anspruch 1, ausgewählt aus 3,3-Bis(picolinamidomethyl)oxetan; 4,4-Bis(picolinamidomethyl)tetrahydro-4H-pyran; 2,2-Bis(picolinoylamidomethyl)-1,3-propandiol; N,N'-Bis(picolinoyl)-1,5-diamino-2,2-dimethylpropan; N,N'-Bis(picolinoyl)-1,5-diamino-3,3-dimethylpentan; und 1,1-Bis(picolinamidomethyl)cyclopropan.

5. Verbindung nach Anspruch 1, bei welcher es sich um N,N'-Bis(picolinoyl)-1,3-diamino-2,2-hydroxymethylpropan handelt.

6. Arzneimittel umfassend eine Verbindung nach einem der Ansprüche 1 bis 5 und einen pharmazeutischen Träger oder Exzipienten.

7. Verbindung nach einem der Ansprüche 1 bis 5 zur Verwendung bei der Therapie.

8. Verwendung einer Verbindung nach einem der Ansprüche 1 bis 5 bei der Herstellung eines Medikaments zur Verwendung bei der Stimulierung des myelopoetischen Systems eines Lebewesens.

9. Verwendung einer Verbindung nach einem der Ansprüche 1 bis 5 bei der Herstellung eines Medikaments zur Verwendung bei der Prävention oder Behandlung einer Virus-Pilz- oder Bakterien-Infektion.

10. Verwendung einer Verbindung nach einem der Ansprüche 1 bis 5 bei der Herstellung eines Medikaments zur Verwendung bei der Prävention oder Behandlung einer Sepsis.

11. Verfahren zur Herstellung einer Verbindung nach einem der Ansprüche 1 bis 5, welches umfaßt:
(a) Bisacylierung eines Diamins der Formel (II):
HN(R₂)-Y-(R₂)NH Formel (II)
in der R₂ und Y wie in Formel (I) definiert sind oder Y zusätzlich durch eine geeignete Schutzgruppe geschützt sein kann; mit einer Verbindung der Formel (IV):
R₁-CO₂H Formel (IV)
in der R₁ wie in Formel (I) definiert ist, unter Verwendung eines geeigneten Aktivierungsmittels in einem geeigneten polaren aprotischen Lösungsmittel, gefolgt von der Entfernung jeglicher Schutzgruppen und, falls gewünscht, durch Salzbildung; oder alternativ durch:
(b) Umwandlung der Säure der Formel (IV) in ein Sätlrehalogenid:
R₁COX'
wobei R₁ wie in Formel (I) definiert ist und X' ein Halogenatom bedeutet, das mit dem Diamin der Formel (II) umgesetzt wird:
HN(R₂)-Y-(R₂)NH Formel (II)
in der R₂ und Y wie in Formel (I) definiert sind oder Y zusätzlich durch eine geeignete Schutzgruppe geschützt sein kann; in Gegenwart einer geeigneten Base, in einem geeigneten Lösungsmittel, gefolgt von der Entfernung jeglicher Schutzgruppen und, falls gewünscht, durch Salzbildung.

## Revendications

1. Composés répondant à la formule (I) 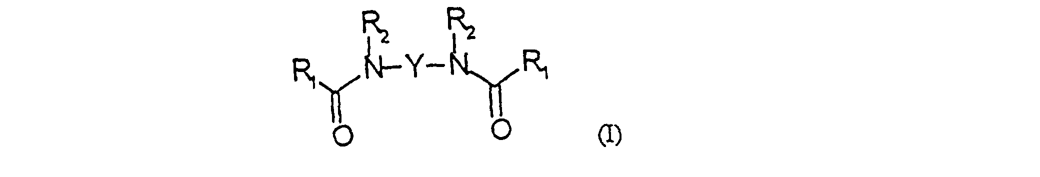 dans laquelle :
R₁ représente, indépendamment, un système de noyau hétérocyclique mono- ou bicyclique tétra- à décagonal contenant jusqu'à 4 hétéroatomes N, O, S dans le noyau, au moins un hétéroatome étant un atome de N, et le noyau étant substitué ou non substitué avec un ou deux groupes alkyle en C₁ à C₄, F, Cl, Br, I, alkoxy en C₁ à C₄, (CH₂)ₘR₆, oxo, oxime, O-(alkyle en C₁ à C₄) oxime, hydroxy, N(R₅)₂, acylamino ou aminoacyle, les systèmes de noyaux monocycliques octo-, nona- et décagonaux étant exclus ;
R₂ représente, indépendamment, l'hydrogène, un groupe (alkyle en C₁ à C₄) (O) R₆ ou alkyle en C₁ à C₄, ou bien R₂ représente un groupe benzyle qui est facultativement substitué avec un ou deux substituants alkyle en C₁ à C₄, alkoxy en C₁ à C₄, F, Cl, I, Br, OH ou N(R₅)₂ ;
Y représente un groupe (CH₂)ₙ, dans lequel au moins un atome de carbone est gem-substitué avec R₃ et R₄ ; R₃ et R₄ représentent, indépendamment, un groupe alkyle en C₁ à C₄, alcényle en C₂ à C₄ ou alcynyle en C₂ à C₄ ; tous ces groupes pouvant être substitués avec un ou deux substituants alkyle en C₁ à C₄, OH, F, Cl, Br, I, N(R₅)₂, (R₅)₂NC(O)-, -(CH₂)ₘR₆, -(CH₂)ₘR₅, -(CH₂)ₘCOR₆ ou -(CH₂)ₘC(O)R₅, ;
ou R₃ et R₄ représentent F, Cl ou Br ;
ou bien R₃ et R₄ peuvent, conjointement, former un noyau cyclique ou hétérocyclique de formule (Ia) : 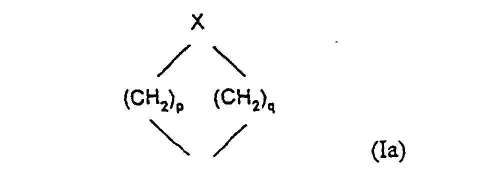
dans laquelle p et q représentent, indépendamment, un nombre entier de 0 à 3 ;
X représente O, S, un groupe CH₂ ou N(R₅) ;
R₅ représente, indépendamment, l'hydrogène, un groupe alkyle en C₁ à C₄ ou benzyle ;
R6 représente, indépendamment, un groupe OR₅, N(R₅)₂ ou SR₅ ; et
n représente un nombre entier de 3 à 8 ;
m représente un nombre entier de 0 à 4 ;
ou leurs sels pharmaceutiquement acceptables ;
sous réserve que : p et q ne soient pas l'un et l'autre égaux à 0 ; et, lorsque R₃ et R₄ représentent F, Cl ou Br, ils ne soient pas présents sur un atome de carbone adjacent à l'atome d'azote,
et à l'exclusion du N5,N5'- ou N4,N4'-(2,2-diméthyl-1,3-propanediyl)bis[1-méthyl-1H-imidazole-4,5-dicarboxamide].

2. Composés suivant la revendication 1, dans lesquels R1 représente un groupe pyridinyle, pyrimidinyle, pyrazinyle, pyridazinyle, quinolinyle, tétrahydroquinolinyle ou pyrrolidinyle ; n a une valeur de 3 à 5 ; R₃ et R₄ représentent des groupes alkyle en C₁ à C₄, substitués avec des substituants alkyle en C₁ à C₄, OH, N(R₅)₁₋₂, -(CH₂)ₘR₆ ou -(CH₂)ₘC(O)R₅ ; ou bien R₃ et R₄ peuvent, conjointement, former un noyau cyclique ou hétérocyclique de formule (Ia) dans laquelle X représente O, S ou un groupe CH₂ ; et p et q ont des valeurs de 1 à 3 ; R₅ représente l'hydrogène ou un groupe alkyle en C₁ à C₄ ; et R₆ représente un groupe OR₅ ou N (R₅)₁₋₂.

3. Composés suivant la revendication 2, dans lesquels R₁ représente un groupe 2-pyridinyle, 2-pyrimidinyle, 2-pyrazinyle, 2-pyrrolidone-5-yle ou 2-pyrrolidinyle ; n est égal à 3 ou 5 ; R₃ et R₄ représentent des groupes alkyle en C₁ ou C₂, substitués avec des substituants alkyle en C₁ ou C₂, OH, N(R₅)₁₋₂ ou -(CH₂)ₘC(O)R₅ ; ou bien R₃ et R₄ peuvent, conjointement, former un noyau hétérocyclique de formule (Ia) dans laquelle X représente O ; et p et q sont égaux à 1 ou 2 ; R₅ représente l'hydrogène ou un groupe alkyle en C₁ à C₄ ; et R₆ représente un groupe OR₅ ou N(R₅)₁₋₂.

4. Composés suivant la revendication 1, choisis dans le groupe consistant en le 3,3-bis(picolinamidométhyl)-oxéthanne ; le 4,4-bis(picolinamidométhyl)tétrahydro-4H-pyranne ; le 2,2-bis(picolinoylamidométhyl)-1,3-propanediol ; le N,N'-bis(picolinoyl)-1,5-diamino-2,2-diméthylpropane ; le N,N'-bis(picolinoyl)-1,5-diamino-3,3-diméthylpentane et le 1,1-bis(picolinamidométhyl)cyclopropane.

5. Composé suivant la revendication 1, qui est le N,N'-bis (picolinoyl)-1,3-diamino-2,2-hydroxyméthylpropane.

6. Composition pharmaceutique comprenant un composé suivant l'une quelconque des revendications 1 à 5 et un support ou excipient pharmaceutique.

7. Composé suivant l'une quelconque des revendications 1 à 5, destiné à être utilisé en thérapeutique.

8. Utilisation d'un composé suivant l'une quelconque des revendications 1 à 5 dans la production d'un médicament destiné à être utilisé dans la stimulation du système myélopoïétique d'un animal.

9. Utilisation d'un composé suivant l'une quelconque des revendications 1 à 5 dans la production d'un médicament destiné à être utilisé dans la prévention ou le traitement d'une infection virale, fongique ou bactérienne.

10. Utilisation d'un composé suivant l'une quelconque des revendications 1 à 5 dans la production d'un médicament destiné à être utilisé dans la prévention ou le traitement de la septicémie.

11. Procédé pour la préparation d'un composé suivant l'une quelconque des revendications 1 à 5, qui comprend :
(a) la bisacylation d'une diamine de formule (II) :
HN(R₂)-Y-(R₂)NH Formule (II)
dans laquelle R₂ et Y répondent aux définitions mentionnées pour la formule (I), ou bien Y peut en outre être protégé par un groupe protecteur convenable ; avec un composé de formule (IV) :
R₁-CO₂H Formule (IV)
dans laquelle R₁ répond à la définition mentionnée pour la formule (I), en utilisant un activateur convenable dans un solvant aprotique polaire convenable, avec ensuite l'élimination de tous les groupes protecteurs et, si cela est désiré, la formation d'un sel ; ou bien, en variante, la transformation de l'acide de formule (IV) en un halogénure d'acide :
R₁COX'
dans lequel R₁ répond à la définition mentionnée pour la formule (I) et X' représente un atome d'halogène,
qui est amené à réagir avec la diamine de formule (II)
HN(R₂)-Y-(R₂)NH Formule (II)
dans laquelle R₂ et Y répondent aux définitions mentionnées pour la formule (I) ou bien Y peut être en outre protégé par un groupe protecteur convenable ; en présence d'une base convenable, dans un solvant convenable, avec ensuite l'élimination de tous les groupes protecteurs et, si cela est désiré, la formation d'un sel.
